# EUROPÄISCHE PATENTANMELDUNG

(11) **EP 2 111 880 A2**
(43) Veröffentlichungstag der Anmeldung: **28.10.2009**
(21) Anmeldenummer: 09156299.1
(22) Anmeldetag: 26.03.2009
(51) Int. Cl.: A61L 31/10, A61L 31/14, A61L 31/02

(54) **Biodegradierbarer Metallstent**

(30) Priorität: 24.04.2008 DE 102008020415
(71) Anmelder: Biotronik VI Patent AG, 6341 Baar (CH)
(72) Erfinder: Adden, Nina, 90427, Nürnberg (DE)
(74) Vertreter: Lindner-Vogt, Karin L.

(57) **Zusammenfassung**

Die vorliegende Erfindung betrifft einen biodegradierbaren Metallstent, ein Verfahren zur Herstellung des erfindungsgemäßen Stents, eine Verwendung der Erfindung zur Verzögerung der Degradierung des erfindungsgemäßen Stents nach Implantation in einen menschlichen oder tierischen Organismus sowie ein Verfahren zur Verzögerung der Degradierung eines erfindungsgemäßen biodegradierbaren Metallstents nach Implantation in einen menschlichen oder tierischen Organismus.

## Beschreibung

### Gebiet der Erfindung:

Die vorliegende Erfindung betrifft einen biodegradierbaren Metallstent, ein Verfahren zur Herstellung des erfindungsgemäßen Stents, eine Verwendung der Erfindung zur Verzögerung der Degradierung des erfindungsgemäßen Stents nach Implantation in einen menschlichen oder tierischen Organismus sowie ein Verfahren zur Verzögerung der Degradierung eines erfindungsgemäßen biodegradierbaren Metallstents nach Implantation in einen menschlichen oder tierischen Organismus.

Stents im Allgemeinen sind endovaskuläre (periphere oder koronare) Prothesen bzw. Implantate, die beispielsweise zur Behandlung von Stenosen verwendet werden. Stents sind außerdem bekannt für die Behandlung von Aneurysmen.

Stents weisen grundsätzlich eine Tragstruktur auf, die geeignet ist, die Wand eines Gefäßes in geeigneter Weise abzustützen, um so das Gefäß zu weiten bzw. ein Aneurysma zu überbrücken. Stents werden dazu in einem komprimierten Zustand in das Gefäß eingeführt und dann an dem zu behandelnden Ort aufgeweitet und gegen die Gefäßwand gedrückt. Dieses Aufweiten kann beispielsweise mit Hilfe eines Ballonkatheters erfolgen. Alternativ sind auch selbstexpandierende Stents bekannt. Diese sind beispielsweise aus einem superelastischem Metall, wie Nitinol, aufgebaut.

Stents werden derzeit in zwei Grundtypen eingeteilt, die dauerhaften Stents und die biodegradierbaren Stents. Dauerhafte Stents sind so ausgestaltet, dass sie im Gefäß für einen unbestimmten Zeitraum verbleiben können. Biodegradierbare Stents hingegen werden über einen vorbestimmten Zeitraum hinweg in einem Gefäß abgebaut. Vorzugsweise werden biodegradierbare Stents erst abgebaut, wenn das traumatisierte Gewebe des Gefäßes verheilt ist und somit der Stent nicht weiter im Gefäßlumen verbleiben muss.

Als biodegradierbare Stentmaterialien sind beispielsweise biodegradierbare Metalllegierungen, Polymere oder Verbundwerkstoffe bekannt, die eine ausreichende strukturelle Tragfähigkeit aufweisen, um das Gefäßlumen über einen vorbestimmten Zeitraum stützen zu können.

Es hat sich allerdings gezeigt, dass durch die Einbringung von und das Verbleiben der Stents in Gefäßsysteme regelmäßig (Spät-)Komplikationen auftreten können, wie z. B. (In-Stent)Restenosen, Gefäßentzündungen und Thrombosen.

Aus diesem Grund wurden Stents dahingehend weiterentwickelt, zusätzlich zu Ihrer Tragstruktur ein oder mehrere Wirkstoffe zu umfassen, die während und/oder nach Implantation des Stents direkt und lokal an den menschlichen oder tierischen Organismus abgegeben werden, um so die Komplikationsrate zu verringern.

Üblicherweise wird ein Wirkstoff mittels eines Polymerträgers auf die Oberfläche des Stentgrundkörpers aufgebracht, wobei der Wirkstoff nach Implantation mittels Diffusions-und/oder Erosionsprozesse aus der Polymermatrix freigesetzt wird.

Wenn ein nicht-degradierbares Polymer als Polymerträger verwendet wird, so kann dies, da das Polymer als Fremdkörper in dem Organismus verbleibt, das Risiko einer Thrombose erhöhen.

Wenn degradierbare Polymere (z. B. Polyester) als Polymerträger verwendet werden, so können die bei der Degradierung entstehenden Polymerabbauprodukte Gefäßentzündungen bis hin zu Gewebsnekrosen hervorrufen. Andererseits können die entstehenden Polymerabbauprodukte auch direkt auf einen biodegradierbaren Metallstentgrundkörper einwirken. Aufgrund des vornehmlich sauren Charakters der Polymerenabbauprodukte (Milchsäure, Glycolsäure, etc.) senkt sich der pH-Wert in der direkten Umgebung des biodegradierbaren Metallstents. Durch die pH-Wert Verschiebung ins Saure kann insbesondere die Zersetzungsgeschwindigkeit eines biodegradierbaren Metallstentgrundkörpers negativ beeinflusst werden.

Als Alternative zu einer Wirkstoffbeschichtung mittels Polymerträger wurden bei Medizinprodukten insbesondere wirkstoffhaltige Öl- und Fettbeschichtungen untersucht (WO 03/039612, WO 2005/053767 und WO 2006/036983). Es wurde gefunden, dass diese Trägermaterialien nach Implantation zwar keine wesentlichen negativen physiologischen Reaktionen im menschlichen oder tierischen Organismus hervorrufen, jedoch haften solche Öl- oder Fettbeschichtungen aufgrund Ihrer mechanischen Eigenschaften zum Teil nicht in einem ausreichenden Maße an der Stentoberfläche.

Um diesen Umstand zu beheben wurden Öle, die üblicherweise flüssig sind, entweder partiell hydriert (WO 2005/053767) oder teilweise vernetzt (WO 2006/036983), um eine ausreichend feste Konsistenz aufzuweisen und so in einem ausreichenden Maße am Stent anhaften zu können.

Aber auch für den Fall, dass Öl- und Fettbeschichtungen in einem ausreichenden Maße an die Stentoberfläche anhaften, weisen solche Beschichtungen in der Praxis üblicherweise immer noch keine ausreichenden mechanischen Eigenschaften auf. Werden beispielsweise Stents vor dem Crimpen auf einen Katheter mit einer Öl- und Fettbeschichtung beschichtet, so weist die Beschichtung nach dem Crimpen Schäden auf.

Zudem weist eine Öl- und Fettbeschichtung üblicherweise eine geringe Kratzfestigkeit auf, so dass das Risiko einer Beschädigung der Beschichtung, beispielsweise durch das Reiben eines Führungskatheters auf der muralen beschichteten Seite eines Stents - d. h. beim typischerweise zylinderförmigen Stent, der äußeren Zylinderfläche, also der dem Gewebe und nicht dem Gefäßlumen zugewandten Fläche - beim Einbringen in den Organismus hoch ist. Aufgrund einer solchen Beschädigung kann das Stentmaterial mit physiologischen Flüssigkeiten in Kontakt kommen, sodass bei einem biodegradierbaren Stent die Degradierung bereits zu diesem Zeitpunkt beginnt. Umfasst die Öl- und Fettbeschichtung zusätzlich ein oder mehrere Wirkstoffe, so kann auch durch die Beschädigung der Beschichtung die freizusetzende Wirkstoffkonzentration nicht mehr ausreichend sein, um die physiologisch erwünschten Wirkungen hervorzurufen.

Es ist deshalb die Aufgabe der vorliegenden Erfindung einen verbesserten biodegradierbaren Metallstent bereitzustellen:
- der einfach herstellbar ist,
- dessen Beschichtung keine unerwünschten physiologischen Reaktionen hervorruft,
- der ausreichende mechanische Eigenschaften aufweist, d. h. dass
   - ein Stent vor dem Crimpen besichtet werden kann und die Beschichtung nach dem Crimpen funktionstüchtig bleibt und/oder
   - die Beschichtung eine ausreichende Kratzfestigkeit aufweist, damit sie insbesondere durch einen Führungskatheter beim Einbringen in den menschlichen oder tierischen Organismus nicht beschädigt wird und/oder
- dessen Abbau gegenüber mit Polymerbeschichteten biodegradierbaren Metallstents verzögert einsetzt.

### Zusammenfassung der Erfindung

Die vorliegende Aufgabe wird durch die Gegenstände der unabhängigen Ansprüche gelöst.

Demnach betrifft ein erster Erfindungsgegenstand einen biodegradierbaren Metallstent, der dadurch gekennzeichnet ist, dass die Oberfläche des Stents mit einer Wachsschicht beschichtet ist.

Ein zweiter Erfindungsgegenstand betrifft ein Verfahren zur Herstellung eines beschichteten biodegradierbaren Metallstents, **dadurch gekennzeichnet, dass** das Verfahren folgende Schritte umfasst oder daraus besteht:
a) Bereitstellen eines Wachses oder einer Mischung aus zwei oder mehreren Wachsen,
b) Herstellen (i) einer Schmelze oder (ii) einer Lösung mit ein oder mehreren Lösungsmitteln des Waches oder der Wachsmischung aus Schritt a) und
c) Bereitstellen eines biodegradierbaren Metallstents und
d) Beschichten der Oberfläche des Stents aus Schritt c) mit (i) der Schmelze oder (ii) der Lösung des Waches oder der Wachsmischung aus Schritt b).

Ein dritter Erfindungsgegenstand betrifft eine Verwendung einer Wachsbeschichtung auf der Oberfläche eines biodegradierbaren Metallstents zur Verzögerung der Degradierung des Stents nach Implantation in einen menschlichen oder tierischen Organismus.

Ein vierter Erfindungsgegenstand betrifft ein Verfahren zur Verzögerung der Degradierung eines biodegradierbaren Metallstents nach Implantation in einen menschlichen oder tierischen Organismus, **dadurch gekennzeichnet, dass** der Stent mit einer Wachsschicht beschichtet wird.

Bevorzugte Ausgestaltungen der erfindungsgemäßen Gegenstände werden in den abhängigen Ansprüchen sowie der nachfolgenden detaillierten Beschreibung dargestellt und sind, sofern sinnvoll, untereinander kombinierbar.

### Detaillierte Beschreibung der Erfindung

Die vorliegende Erfindung beruht auf der Erkenntnis, dass aufgrund der Auswahl von ein oder mehreren Wachsen als Beschichtungsmaterial ein erfindungsgemäß beschichteter biodegradierbarer Metallstent bereitgestellt wird, der eine oder mehrere der vorstehenden Aufgaben löst.

Eine erfindungsgemäße Wachsbeschichtung weist im Vergleich zu einer entsprechenden Öl- oder Fettbeschichtung eine höhere Kratzfestigkeit auf, so dass beispielsweise nach Verwendung eines Führungskatheters beim Einbringen in den menschlichen oder tierischen Organismus die Beschichtung nicht in der Weise beschädigt wird, dass physiologische Flüssigkeiten in der Weise mit dem Stentmaterial in Kontakt kommen, dass eine Degradierung des Stents dadurch beginnt. Mit anderen Worten bleibt die erfindungsgemäße Wachsschicht nach dem Einbringen in den menschlichen oder tierischen Körper mittels Führungskatheter funktionstüchtig.

Weiterhin ist die erfindungsgemäße Wachsschicht im Vergleich zu einer Öl- und Fettbeschichtung besser dafür geeignet, die Oberfläche eines Stents vor dem Crimpen zu beschichten, da auch nach dem Crimpen die erfindungsgemäße Wachsbeschichtung funktionstüchtig bleibt.

Für den Fall, dass gemäß einer bevorzugten Ausgestaltung die Wachsschicht ein oder mehrere Wirkstoffe enthält, besteht ein weiterer Vorteil darin, dass durch die verbesserten mechanischen Eigenschaften das Risiko, dass nach Implantation die Beschichtung keine effektiven Wirkstoffkonzentration bzw. -freisetzung aufweist, reduziert wird.

Da die erfindungsgemäße Wachsbeschichtung nach Implantation in den menschlichen oder tierischen Körper funktionstüchtig bleibt, hydrophob ist und insbesondere bei Kontakt mit physiologischen Flüssigkeiten nicht quillt, wird die Degradierung eines biodegradierbaren Metallstents im Vergleich zu einem biodegradierbaren Metallstent mit einer Polymerschicht verzögert, da die Polymerbeschichtung üblicherweise bei Kontakt mit physiologischen Flüssigkeiten quillt. Vorteil einer verzögerten Degradierung besteht darin, dass der Stentgrundkörper über einen längeren Zeitraum seine Integrität nicht verliert und so die Abstützung im Gefäß über einen längeren Zeitraum gewährleisten kann.

Da die erfindungsgemäße Wachsschicht des Weiteren im Vergleich zu einer degradierbaren Polymerschicht keine sauren Abbauprodukte liefert wird durch die erfindungsgemäße Wachsbeschichtung die Zersetzungsgeschwindigkeit des biodegradierbaren Metallstentgrundkörpers im Weiteren nicht negativ beeinflusst.

Des Weiteren weist die erfindungsgemäße Wachsschicht im implantierten Zustand auch keine unerwünschten physiologischen Nebenwirkungen auf, so dass das Risiko einer (Spät-)Komplikation weiter vermindert wird.

Im Übrigen ist der erfindungsgemäße biodegradierbare Metallstent mit Wachsschicht einfach herstellbar, da übliche Wachse, die für pharmazeutische Zwecke geeignet sind, zum einen kommerziell erhältlich sind und diese zum anderen lediglich in einem geeigneten Lösungsmittel gelöst werden müssen, damit der Stent beschichtet werden kann. Hydrierungsschritte bzw. Vernetzungsschritte, wie sie bei Öl- und Fettbeschichtungen üblicherweise notwendig sind, sind für die Herstellung der erfindungsgemäßen Wachsbeschichtung nicht notwendig.

Im Sinne der vorliegenden Erfindung bedeuten die Begriffe "biodegradierbarer Metallstent" bzw. "biodegradierbarer Stent", dass der Grundkörper des Metallstents in physiologischer Umgebung, insbesondere im Gefäßsystem eines menschlichen oder tierischen Körpers degradiert, d. h. so abgebaut wird, dass der Stent seine Integrität verliert. Vorzugsweise degradiert der biodegradierbare Metallstentgrundkörper erst dann, wenn die Funktion des Stents nicht mehr physiologisch sinnvoll bzw. notwendig ist. Bei biodegradierbaren Metallstents bedeutet das, dass der Stent vorzugsweise erst dann degradiert oder seine Integrität verliert, wenn das traumatisierte Gewebe des Gefäßes verheilt ist und der Stent somit nicht länger im Gefäßlumen verbleiben muss.

Gemäß der vorliegenden Erfindung umfasst oder besteht der biodegradierbare Metallstent aus einem metallischen Werkstoff, der eine biokorrodierbare Legierung darstellt, wobei die Hauptkomponente der Legierung ausgewählt wird aus der Gruppe Magnesium, Eisen, Zink und Wolfram; insbesondere wird für einen degradierbaren metallischen Werkstoff eine Magnesiumlegierung bevorzugt.

Die Legierung, insbesondere umfassend Magnesium, Eisen, Zink und/oder Wolfram, ist so in ihrer Zusammensetzung zu wählen, dass sie biokorrodierbar ist. Als biokorrodierbar im Sinne der vorliegenden Erfindung werden Legierungen bezeichnet, bei denen in physiologischer Umgebung ein Abbau stattfindet, der letztendlich dazu führt, dass der gesamte Stent oder der aus dem Werkstoff gebildete Teil des Stents seine mechanische Integrität verliert. Unter Legierung wird vorliegend ein metallisches Gefüge verstanden, deren Hauptkomponente Magnesium, Eisen, Zink oder Wolfram ist. Hauptkomponente ist die Legierungskomponente, deren Gewichtsanteil an der Legierung am Höchsten ist. Ein Anteil der Hauptkomponente beträgt vorzugsweise mehr als 50 Gew.%, weiter bevorzugt mehr als 70 Gew.%. Eine Magnesiumlegierung ist bevorzugt.

Ist der Werkstoff eine Magnesiumlegierung, so enthält diese vorzugsweise Yttrium und weitere Seltenerdmetalle, da sich eine derartige Legierung aufgrund ihrer physikochemischen Eigenschaften und ihrer hohen Biokompatibilität, insbesondere auch ihrer Abbauprodukte, auszeichnet.

Besonders bevorzugt werden Magnesiumlegierungen der WE-Reihe, insbesondere WE43 sowie Magnesiumlegierungen der Zusammensetzung Seltenerdmetalle 5,5 - 9,9 Gew.%, davon Yttrium 0,0 - 5,5 Gew.% und Rest < 1 Gew.%, der Zirkonium und/oder Silizium enthalten kann, wobei Magnesium den auf 100 Gew.% fehlenden Anteil an der Legierung einnimmt, eingesetzt. Diese Magnesiumlegierungen bestätigten bereits experimentell und in ersten klinischen Versuchen ihre besondere Eignung, d. h. sie zeigen eine hohe Biokompatibilität, günstige Verarbeitungseigenschaften, gute mechanische Kennwerte und ein für die Einsatzzwecke adäquates Korrosionsverhalten. Unter der Sammelbezeichnung "Seltenerdmetalle" werden vorliegend Scandium (21), Yttrium (39), Lanthan (57) und die 14 an Lanthan (57) folgenden Elemente, nämlich Cer (58), Neodym (60), Promethium (61), Samarium (62), Europium (63), Gadolinium (64), Terbium (65), Dysprosium (66), Holmium (67), Erbium (68), Thulium (69), Ytterbium (70) und Lutetium (71), verstanden.

Im Sinne der vorliegenden Erfindung können als erfindungsgemäße biodegradierbare Metallstents alle üblichen Stentgeometrien verwendet werden. Insbesondere bevorzugt sind Stentgeometrien, die insbesondere in US 6,896,695, US 2006/241742, US 5,968,083 (Tenax), EP 1 430 854 (Helix-Design), US 6,197,047 und EP 0 884 985 beschrieben werden.

Im Sinne der vorliegenden Erfindung bedeutet der Begriff "Wachsschicht" oder "Wachsbeschichtung", dass die Oberfläche des biodegradierbaren Metallstents vollständig oder teilweise mit einer Schicht umfassend oder bestehend aus ein, zwei oder mehreren Wachsen beschichtet ist.

Vorzugsweise ist die Oberfläche des erfindungsgemäßen Metallstents sowohl auf der luminalen Seite, d. h. beim typischerweise zylinderförmigen Stent, die Oberfläche in Richtung der mittigen Zylinderachse, als auch auf der muralen Seite, d. h. beim typischerweise zylinderförmigen Stent, die äußere Zylinderfläche, mit der erfindungsgemäßen Wachsschicht beschichtet. In einer besonders bevorzugten Ausgestaltung ist die erfindungsgemäße Stentoberfläche nur auf der muralen Seite mit der Wachsschicht beschichtet, so dass die Degradierung des erfindungsgemäßen Stents von der luminalen Seite, nicht aber von der muralen Seite her beginnt.

Der Begriff "Wachs" ist im Sinne der vorliegenden Erfindung eine Sammelbezeichnung für eine Reihe natürlicher (pflanzlicher, tierischer oder mineralischer Herkunft) oder künstlich gewonnener Stoffe, die im Allgemeinen bei Raumtemperatur dehnbar sind, oberhalb 45°C ohne Zersetzen schmelzen, im geschmolzenen Zustand eine geringe Viskosität aufweisen und in Wasser unlöslich sowie hydrophob sind. Üblicherweise sind Wachse Gemische von Estern höherer linearer Fettsäuren (C₁₈ bis C₃₄ und mehr) mit höheren (meist gleich langen 1-wertigen) Alkoholen. So sind beispielsweise Palmitate, Palmitoleate, Hydroxypalmitate und Oleatester von langen Alkoholen (C₃₀ bis C₃₂) die Hauptbestandteile von Bienenwachs. Geringe Anteile an freien Säuren und Alkoholen ähnlicher Länge können ebenfalls enthalten sein.

Geeignete Wachse im Sinne der vorliegenden Erfindung werden üblicherweise ausgewählt aus der Gruppe der natürlichen oder künstlichen Wachse, die insbesondere für pharmazeutische Zwecke und hier insbesondere zur Implantation in einen menschlichen oder tierischen Körper verwendet werden können, d. h. pharmazeutische Qualität gemäß Ph. Eur. 5 (European Pharmacopeia 5).

Vorzugsweise umfasst oder besteht die Wachsschicht aus ein, zwei oder mehrere natürlichen Wachsen. Besonders bevorzugt werden die natürlichen Wachse ausgewählt aus der Gruppe bestehend aus:
- tierischen Wachsen: gebleichtes Wachs (Cera alba), Bienenwachs (Cera flava) und Lanolin (Wollfett bzw. Adeps Lanae) und/oder
- pflanzlichen Wachsen: Simmondsia-Wachs (Jojobaöl), Carnaubawachs (Brazil wax) und Candelillawachs (Kanutillawachs).

Bienenwachs ist insbesondere wegen seiner Dehnbarkeit bevorzugt, wohingegen Carnaubawachs insbesondere wegen seiner Kratzfestigkeit bevorzugt wird, was eine Beschichtung des Stents vor dem Crimpen ermöglicht. Des Weiteren ist Candellilawachs härter als Bienenwachs, jedoch weicher als Carnaubawachs.

In einer weiteren bevorzugten Ausgestaltung können durch das Mischen von zwei oder mehreren Wachsen die gewünschten mechanischen Eigenschaften den erforderlichen Bedürfnissen besser angepasst werden. Bevorzugte Gewichtsverhältnisse einer Mischung aus zwei der erfindungsgemäßen Wachse liegen bei 1:99 bis 99:1, weiter bevorzugt bei 90:10 bis 10:90, besonders bevorzugt 80:20 bis 20:80 und ganz besonders bevorzugt bei 70:30 bis 30:70. Ganz besonders bevorzugt wird hierfür eine Mischung aus Bienenwachs und Carnaubawachs verwendet.

In einer weiteren bevorzugten Ausgestaltung können durch das Zumischen von freien Fettsäuren die gewünschten mechanischen Eigenschaften den erforderlichen Bedürfnissen besser angepasst werden. Geeignete Fettsäuren können bevorzugt ausgewählt werden aus der Gruppe bestehend aus: gesättigte Fettsäuren wie Buttersäure, Valeriansäure, Capronsäure, Önansäure, Caprylsäure, Pelargonsäure, Caprinsäure, Laurinsäure, Myristinsäure, Palmitinsäure, Margarinsäure, Stearinsäure, Arachinsäure, Behensäure; einfach ungesättigte Fettsäuren wie Palmitoleinsäure, Ölsäure, Elaidinsäure, Vaccensäure, Icosensäure, Cetoleinsäure; oder mehrfach ungesättigte Fettsäuren wie Linolsäure, Arachindonsäure, Timnodonsäure, Clupandonsäure, Cervonsäure. Besonders bevorzugt sind gesättigte Fettsäuren wie Caprylsäure, Pelargonsäure, Caprinsäure, Laurinsäure, Myristinsäure, Palmitinsäure.

In einer weiteren bevorzugten Ausgestaltung kann der Stentgrundkörper mit einer Fettsäurelösung als Haftvermittler vorbehandelt werden, um ein besseres Anhaften des Wachses an den Stent zu erreichen. Geeignete Fettsäurelösungen als Haftvermittler umfassen oder bestehen bevorzugt aus gesättigten Fettsäuren wie Buttersäure, Valeriansäure, Capronsäure, Önansäure, Caprylsäure, Pelargonsäure, Caprinsäure, Laurinsäure, Myristinsäure, Palmitinsäure, Margarinsäure, Stearinsäure, Arachinsäure oder Behensäure. Besonders bevorzugt kann hierfür Laurinsäure verwendet werden.

In einer weiteren bevorzugten Ausgestaltung umfasst oder besteht die Wachsschicht zusätzlich aus ein, zwei oder mehreren Wirkstoffen.

Ein Wirkstoff im Sinne dieser Erfindung ist eine Substanz oder eine Verbindung, die im menschlichen oder tierischen Körper eine biologische Reaktion hervorruft. In diesem Sinne kann Wirkstoff auch synonym zu Arzneistoff und/oder Pharmakon verwendet werden. Im Sinne der vorliegenden Erfindung ist der erfindungsgemäße Stent mit ein oder mehreren Wirkstoffen in einer Konzentration beschichtet, die ausreicht, die gewünschten physiologischen Reaktionen hervorzurufen.

Erfindungsgemäß zu verwendende Wirkstoffe werden bevorzugt ausgewählt aus der Gruppe bestehend aus: Antiphlogistika, vorzugsweise Dexamethason, Methylprednisolon und Diclophenac; Cytostatika, vorzugsweise Paclitaxel, Colchicin, Actinomycin D und Methotrexat; Immunsuppressiva, vorzugsweise Limus-Verbindungen, weiter bevorzugt Sirolimus (Rapamycin), Zotarolimus (Abt-578), Tacrolimus (FK-506), Everolimus, Biolimus, insbesondere Biolimus A9 und Pimecrolimus, Cyclosporin A und Mycophenolsäure; Thrombozytenaggregationshemmer, vorzugsweise Abciximab und Iloprost; Statine, vorzugsweise Simvastatin, Mevastatin, Atorvastatin, Lovastatin, Pitavastatin und Fluvastatin; und Estrogene, vorzugsweise 17b-Estradiol, Daizein und Genistein; Lipidregulatoren, vorzugsweise Fibrate; Immunsuppressiva; Vasodilatatoren, vorzugsweise Satane; Calciumkanalblocker; Calcineurininhibitoren, vorzugsweise Tacrolimus; Antünflammatorika, vorzugsweise Imidazole; Antiallergika; Oligonucleotide, vorzugsweise Decoy-Oligodesoxynukleotid (dODN); Endothelbildner, vorzugsweise Fibrin; Steroide; Proteine/Peptide; Proliferationshemmer; Analgetika und Antirheumatika.

Besonders bevorzugt werden erfindungsgemäß Paclitaxel und Limus-Verbindungen, weiter bevorzugt Sirolimus (Rapamycin), Zotarolimus (Abt-578), Tacrolimus (FK-506), Everolimus, Biolimus, insbesondere Biolimus A9 und Pimecrolimus, ganz besonders bevorzugt Rapamycin (Sirolimus) als der oder die weiteren Wirkstoffe verwendet.

In einer besonders bevorzugten Ausgestaltung ist der erfindungsgemäße Stent nur auf der muralen Oberflächenseite des Stents vollständig oder teilweise, bevorzugt vollständig mit der wirkstoffhaltigen Wachsschicht beschichtet. Diese bevorzugte wirkstoffhaltige Beschichtung hat zum einen den Vorteil, dass der oder die Wirkstoffe direkt an den Zielort des Gewebes abgegeben werden und keine bzw. nicht relevante systemische Nebenwirkungen entfalten. Zum anderen weist eine dementsprechende wirkstoffhaltige Beschichtung gegenüber einer Beschichtung sowohl auf der luminalen als auch muralen Seite ein vermindertes Risiko einer Thrombose auf, da bei gleichzeitiger wirkstoffhaltiger Beschichtung auf der luminalen und muralen Seite von Stents beobachtet wurde, dass die Endothelialisierung eines in dieser Weise beschichteten Stents (Bewachsen des Stents mit Gefäßzellen) verlangsamt bis verhindert wird und damit das Thromboserisiko erhöht ist.

In einer weiteren bevorzugten erfindungsgemäßen Ausgestaltung kann der erfindungsgemäße Stent zusätzlich zu einer muralen wirkstoffhaltigen Beschichtung eine weitere erfindungsgemäße Wachsschicht ohne Wirkstoff auf der luminalen Oberflächenseite aufweisen. Eine solche Beschichtung hat den Vorteil, dass, sofern erwünscht, die Degradierung des Stents (weiter) verzögert wird.

In einer weiteren bevorzugten Ausgestaltung kann ein erfindungsgemäß beschichteter Stent zusätzlich ein oder mehrere weitere Beschichtungen als sogenannte "Topcoats" aufweisen, die vollständig oder teilweise auf der Oberfläche der Wachsschicht beschichtet sind. Solche "Topcoats" können wirkstofffrei sein oder ein oder mehrere Wirkstoffe umfassen.

In einer bevorzugten Ausgestaltung weist ein erfindungsgemäßer Stent eine wirkstofffreie Wachsschicht sowie einen "Topcoat" mit einem oder mehreren Wirkstoffen auf. Hierbei übernimmt die Wachsschicht die Funktion der Degradationskontrolle und schützt zusätzlich das in dem "Topcoat" enthaltenen Wirkstoff vor Abbauprodukten des Stentgrundkörpers.

In dem Fall, dass ein erfindungsgemäßer Stent mit einer wirkstoffhaltigen Wachsschicht beschichtet ist, übernimmt ein bevorzugt wirkstofffreier "Topcoat" die Funktion, dass die Abrasion der Wirkstoffbeschichtung der Wachsschicht zusätzlich zu den bereits vorhandenen Materialeigenschaften der erfindungsgemäßen Wachsschicht verringert wird.

Als "Topcoat" können die gleichen Materialien bzw. bevorzugten Ausgestaltungen der erfindungsgemäßen "Wachsschicht" verwendet werden. Alternativ oder kumulativ können ein, zwei oder mehrere übliche Polymere umfasst sein ausgewählt aus der Gruppe bestehend aus:
- nicht degradierbare Polymere: Polyethylen; Polyvinylchlorid; Polyacrylate; vorzugsweise Polyetyl- und Polymethylacrylate, Polymethylmetacrylat, Polymethyl-co-ethylacrylat und Ethylen/Ethylacrylat; Polytetrafluorethylen, vorzugsweise Ethylen/Chlortrifluorethylen Copolymere, Ethylen/Tretrafluorethylen Copolymere; Polyamide, vorzugsweise Polyamidimid, PA-11, PA-12, PA-46, PA-66; Polyetherimid; Polyethersulfon; Poly(iso)butylen; Polyvinylchlorid; Polyvinylfluorid; Polyvinylalkohol; Polyurethan; Polybuthylenterephthalat; Silikone; Polyphosphazen; Polymerschäume, vorzugsweise Polymerschäume aus Carbonaten, Styrolen; Copolymere und/oder Blends der aufgezählten Polymerklassen, Polymere der Klasse der Thermoplaste sowie
- degradierbare Polymere: Polydioxanon; Polyglycolid; Polycaprolacton; Polylactide, vorzugsweise Poly-L-Lactid, Poly D,L Lactid, und Copolymere sowie Blends hiervon, vorzugsweise Poly(L-Lactid-co-glycolid), Poly(D,L-lactid-co-glycolid), Poly(L-Lactid-co-D,L-Lactid), Poly(I-Lactid-co-trimethylen carbonat); Triblockcopolymere; Polysaccharide, vorzugsweise Chitosan, Levan, Hyaluronsäure, Heparin, Dextran, Cellulose; Polyhydroxyvalerat; Polyphosphazen; Polyethylenoxid; Polyphosphorylcholin; Fibrin; Albumin; Polyhydroxybuttersäure, vorzugsweise ataktische, isotaktische und/oder syndiotaktische Polyhydroxybuttersäure sowie deren Blends.

Besonders bevorzugt sind Materialien für einen "Topcoat" aus der Gruppe der Wachse oder der degradierbaren Polymere ausgewählt, ganz besonders bevorzugt wird Carnaubawachs als "Topcoat" verwendet.

Die bevorzugten Ausgestaltungen des erfindungsgemäß verwendbaren Stents können in allen denkbaren Varianten untereinander, aber auch mit den weiteren hierin offenbarten bevorzugten Ausgestaltungen kombiniert werden.

Gemäß des zweiten Erfindungsgegenstandes wird ein Herstellverfahren für wachsbeschichtete Stents, bevorzugt erfindungsgemäße Stents beansprucht.

Die Beschichtung der Oberfläche des erfindungsgemäßen Stents wird gemäß üblicher Verfahren bewerkstelligt. Üblicherweise wird das Wachs oder die Wachsmischung aus zwei, drei oder mehreren Wachsen in einem geeigneten Lösungsmittel gelöst.

Geeignete Lösungsmittel werden bevorzugt ausgewählt aus der Gruppe bestehend aus: Cyclohexan, Chloroform, Aceton, Petrolether, Ethylacetat, Tetrahydrofuran (THF) und Dichlormethan.

Bienenwachs wird vorzugsweise in Cyclohexan, Chloroform und THF gelöst. Carnaubawachs wird bevorzugt in Chloroform und bei 40°C auch in Dichlormethan und Cyclohexan gelöst. Candellilawachs wird bevorzugt in Aceton oder Petrolether gelöst.

Die so erhaltene Wachslösung wird mittels der folgenden üblichen Verfahren auf einen Stent bzw. einen gecrimpten Stent auf einem Katheter ein, zwei oder mehrmals aufgetragen: Tauchverfahren ("Dip-Coating"), Sprühbeschichtung mittels Ein- bzw. Mehrstoffdüse, Rotationszerstäubung und Druckdüsen, Pinseln, Drucken etc.

Gegebenenfalls kann einem oder mehreren Beschichtungsschritten ein üblicher Trocknungsschritt oder andere übliche physikalische oder chemische Nachbearbeitungsschritte, z. B. Vakuum- oder Plasmabehandlung, folgen, bevor der erfindungsgemäße Stent weiter behandelt wird.

Umfasst die erfindungsgemäße Wachsschicht ein oder mehrere Wirkstoffe, so werden der oder die Wirkstoffe in einer ausreichenden Konzentration in der Lösung gelöst oder suspendiert, bevor der Stent oder der gecrimpte Stent auf dem Katheter mit der Lösung/Suspension mittels obiger Verfahren beschichtet wird. Die Konzentration des oder der Wirkstoffe auf dem beschichteten Stent ist üblicherweise so ausgestaltet, dass der oder die Wirkstoffe jeweils oder in Kombination die gewünschte physiologische Reaktion hervorruft.

Für den Fall, dass vorwiegend nur die murale Oberfläche eines erfindungsgemäßen Stents mit einer wirkstoffhaltigen Wachsschicht beschichtet werden soll, kann dies vorzugsweise dadurch bewerkstelligt werden, dass in den vorgenannten Verfahren der Stentgrundkörper auf beispielsweise einen Zylinder, Kanüle oder Dorn aufgesteckt bzw. auf einen Katheter aufgecrimpt wird. Alternativ könnte die abluminale Beschichtung mit weiteren Wirkstoffen über einen Walzenauftrag oder einen selektiven Auftrag durch Bestreichen, Befüllen von Kavitäten vorgenommen werden.

Die Beschichtungsverfahren zur erfindungsgemäßen Wachsschicht können ebenfalls auf den Topcoat angewendet werden.

Die Verwendung und das Verfahren gemäß des dritten und vierten Erfindungsgegenstandes können mit den bevorzugten Ausgestaltungen des erfindungsgemäß verwendbaren Stents in allen denkbaren Varianten ausgeführt werden.

### Ausführungsbeispiele:

Die vorliegende Erfindung wird im Folgenden durch Ausführungsbeispiele beschrieben, die jedoch den Schutzumfang des erfindungsgemäßen Gegenstandes nicht limitieren.

### Ausführungsbeispiel 1: Wachsbeschichtung eines bereits gecrimpten Stents

Ein auf einen Katheter gecrimpter Stent aus der biokorrodierbaren Magnesiumlegierung WE43 (97 Gew.% Magnesium, 4 Gew.% Yttrium, 3 Gew.% Seltenerdmetalle außer Yttrium) wird von Staub und Rückständen gereinigt und wie folgt beschichtet:
Bienenwachs (Fa. Gustav Hees) wird bei 60°C geschmolzen. Die Katheterspitze wird in die Schmelze getaucht und langsam herausgezogen. Nach 1 Minute Trocknungszeit wird der Protector wieder auf den Katheter gezogen.

### Ausführungsbeispiel 2: Wachsbeschichtung eines nicht-gecrimpten Stents

Ein Stent aus der biokorrodierbaren Magnesiumlegierung WE43 (97 Gew.% Magnesium, 4 Gew.% Yttrium, 3 Gew.% Seltenerdmetalle außer Yttrium) wird von Staub und Rückständen gereinigt und in einer Lösung von Laurinsäure in Chloroform für 16 Stunden vorbehandelt.

Eine Wachsmischung wird wie folgt hergestellt: 3 Gew.% einer Mischung aus 80 Gew.% Bienenwachs (Fa. Gustav Hees) und 20 Gew.% Carnaubawachs (Fa. Gustav Hees) werden in Chloroform gelöst, wobei die Gewichtsangaben sich auf die resultierende Mischung beziehen.

Der Stent wird an einem Haken befestigt. Mit Hilfe eines Dipping Systems (Fa. Specialty Coating Systems) wird der Stent unter konstanten Umgebungsbedingungen (Raumtemperatur (RT); 42% Luftfeuchte) in die Wachsmischung getaucht und mit einer Geschwindigkeit von 1 mm pro Minute wieder herausgezogen.

Der Stent wird 5 Minuten bei RT getrocknet. Es sind weitere Tauch-Durchgänge möglich.

Der fertig beschichtete Stent wird für 16 h bei 40°C in einem Vakuumofen (Vakucell; Fa. MMM) getrocknet.

### Ausführungsbeispiel 3: wirkstoffhaltige Wachsbeschichtung eines nicht-gecrimpten Stents

Ein PRO-Kinetic^{®} Stent (Fa. BIOTRONIK) wird in Chloroform gereinigt.

Eine Wachsmischung wird wie folgt hergestellt: 3 Gew.% einer Mischung aus 70 Gew.% Bienenwachs (Fa. Gustav Hees) und 30 Gew.% Carnaubawachs (Fa. Gustav Hees) werden in Chloroform gelöst, wobei die Gewichtsangaben sich auf die resultierende Mischung beziehen. 30 Gew.% Rapamycin bezogen auf den Wachsanteil in der Mischung werden hinzugegeben und verrührt.

Der Stent wird in eine geeignete Stentbeschichtungsapparatur (DES Coater, Eigenentwicklung Fa. Biotronik) eingespannt, so dass eine Hälfte des Stents in der Apparatur eingespannt ist und die andere Hälfte des Stents beschichtet werden kann. Mit Hilfe eines Airbrush Systems (Fa. EFD oder Spraying System) wird der sich drehende Stent unter konstanten Umgebungsbedingungen (Raumtemperatur; 42% Luftfeuchte) auf der Hälfte des Stents, die nicht in die Apparatur eingespannt ist, beschichtet.

Nach Erreichen der beabsichtigten Schichtmasse von ca. 300 µg wird der Stent 5 Minuten bei RT getrocknet, ehe nach Ausspannen des Stents aus der Beschichtungsapparatur, Drehen und erneutem Einspannen der bereits beschichteten Hälfte des Stents die unbeschichtete Seite analog beschichtet wird.

Der fertig beschichtete Stent wird für 16 h bei 60°C in einem Vakuumofen (Vakucell; Fa. MMM) getrocknet.

## Patentansprüche

1. Biodegradierbarer Metallstent **dadurch gekennzeichnet, dass** die Oberfläche des Stents mit einer Wachsschicht beschichtet ist.

2. Stent gemäß Anspruch 1, **dadurch gekennzeichnet, dass** die Wachsschicht ein, zwei oder mehrere natürliche Wachse umfasst oder daraus besteht.

3. Stent gemäß Anspruch 2, **dadurch gekennzeichnet, dass** das oder die natürlichen Wachse ausgewählt werden aus der Gruppe bestehend aus: Gebleichtes Wachs, Bienenwachs, Lanolin, Simmondsia-Wachs, Carnaubawachs und Candelillawachs.

4. Stent gemäß einem der vorstehenden Ansprüche, **dadurch gekennzeichnet, dass** die Wachsschicht ein, zwei oder mehrere Wirkstoffe umfasst.

5. Stent gemäß Anspruch 4, **dadurch gekennzeichnet, dass** die wirkstoffhaltige Wachsschicht auf der muralen Oberfläche des Stents beschichtet ist.

6. Verfahren zur Herstellung eines beschichteten biodegradierbaren Metallstents, **dadurch gekennzeichnet, dass** das Verfahren folgende Schritte umfasst oder daraus besteht:
a) Bereitstellen eines Wachses oder einer Mischung aus zwei oder mehreren Wachsen,
b) Herstellen (i) einer Schmelze oder (ii) einer Lösung mit ein oder mehreren Lösungsmitteln des Waches oder der Wachsmischung aus Schritt a) und
c) Bereitstellen eines biodegradierbaren Metallstents und
d) Beschichten der Oberfläche des Stents aus Schritt c) mit (i) der Schmelze oder (ii) der Lösung des Waches oder der Wachsmischung aus Schritt b).

7. Verwendung einer Wachsbeschichtung auf der Oberfläche eines biodegradierbaren Metallstents zur Verzögerung der Degradierung des Stents nach Implantation in einen menschlichen oder tierischen Organismus.

8. Verfahren zur Verzögerung der Degradierung eines biodegradierbaren Metallstents nach Implantation in einen menschlichen oder tierischen Organismus, **dadurch gekennzeichnet, dass** der Stent mit einer Wachsschicht beschichtet wird.
